# EUROPEAN PATENT APPLICATION

(11) **EP 3 960 850 A1**
(43) Date of publication of application: **02.03.2022**
(21) Application number: 19925757.7
(22) Date of filing: 26.04.2019
(51) Int. Cl.: C12N 7/01, A61K 39/12, A61P 31/20, C12R 1/93

(54) **ATTENUATED AFRICAN SWINE FEVER VIRUS WITH DELETED GENE AND USE OF SAME AS VACCINE**

(71) Applicant: Harbin Veterinary Research Institute, Chinese Academy of Agricultural Sciences, Harbin, Heilongjiang 150069 (CN)
(72) Inventor: BU, Zhigao, Harbin, Heilongjiang 150069 (CN); CHEN, Weiye, Harbin, Heilongjiang 150069 (CN); ZHAO, Dongming, Harbin, Heilongjiang 150069 (CN); HE, Xijun, Harbin, Heilongjiang 150069 (CN); LIU, Renqiang, Harbin, Heilongjiang 150069 (CN); LIU, Jinxiong, Harbin, Heilongjiang 150069 (CN)
(74) Representative: Tiburzi, Andrea
(86) International application number: PCT/CN2019/084469
(87) International publication number: WO 2020/215301

(57) **Abstract**

Provided is an attenuated African swine fever virus, a gene of which has been deleted, which can be used as a vaccine, a vaccine thereof, and a construction method therefor. The construction method comprises using Chinese epidemic strain Pig/CN/HLJ/2018 of African swine fever, and deleting a virulence gene of the African swine fever virus by means of gene engineering technology, thereby obtaining viruses, which have undergone gene deletion, from which MGF360-505R has been deleted and both CD2V and MGF360-505R have been deleted.

## Description

### TECHNICAL FIELD

The present invention relates to the field of veterinary medicine, specifically to prevention and treatment of animal diseases, and more specifically to an attenuated African swine fever virus and a vaccine.

### BACKGROUND

African swine fever virus is the only member of an Asfarviridae family, and mainly replicates in cytoplasm of cells. African swine fever is an acute, severe, and highly contagious infectious disease of domestic pigs and wild boars. The virulent strain can kill domestic pigs within about 5-14 days of infection. The mortality rate is close to 100%. There is no effective preventive vaccine. There is no specific therapeutic drug. Domestic pigs, wild boars and soft ticks at all stages are natural hosts of African swine fever, which can be transmitted directly between domestic pigs and wild boars, or transmitted through tick bites, and also transmitted through virus-contaminated swill, feed, and salted dry ham and other pork products across countries and regions. When an epidemic is discovered, pigs should be culled. African swine fever is one of the most serious infectious diseases that harm pigs in the world and is the number one foreign animal disease that China focuses on preventing.

China's pig breeding accounts for more than 50% of the world. According to the characteristics of the development of African swine fever on a global scale and the status quo of China's pig breeding industry, the African swine fever epidemic will be further expanded in China and will exist for a long time. First, the huge amount of pig breeding in China, the low level of overall breeding, the poor biosafety precautions, the frequent trans-regional transportation of pigs, and the existence of wild hosts provide convenient conditions for the spread of the African swine fever epidemic. Second, the number of epidemic-stricken countries in neighboring countries has increased year by year, and there are natural sources of diseases. Third, according to the history of African swine fever's purification and eradication abroad, once an African swine fever epidemic occurs, it will take a long time and a great price to completely eradicate it. Therefore, it is necessary to accelerate research on the epidemiology of African swine fever, related pathogenic mechanisms, rapid diagnosis and detection technology products, and prevention and control vaccines, and to do a good job in the long-term response to complex African swine fever's prevention and control technology and policy deployment. According to China's national conditions and existing experience in the prevention and control of viral diseases, vaccines are the most effective and economical method to prevent and control African swine fever. Therefore, it is urgent to develop effective and safe vaccines! In particular, the African swine fever epidemic broke out in China in early August 2018. As of January 14, 2019, 24 provinces have had the epidemic, and more than 916,000 pigs have been culled, causing direct economic losses of several billion yuan, and indirect losses were unmeasurable. The African swine fever epidemic has brought huge losses to China's pig breeding industry and national economy. Because of the first occurrence in China, there is a lack of systematic scientific knowledge of the African swine fever and an effective prevention and control technology system. China can only rely on measures such as simple killing and disinfection for prevention and control, which has brought great difficulties to the prevention and control of the epidemic.

Since the discovery of African swine fever virus for more than 100 years, traditional vaccine development strategies [Forman, AJ, RC Wardley, and PJ. Wilkinson, The immunological response of pigs and guinea pigs to antigens of African swine fever virus. Arch Virol, 1982. 74 (2-3): p. 91-100; Mebus, C.AV African swine fever. Adv Virus Res, 1988. 35: p. 251-69; Stone, SS and WR Hess, Antibody response to inactivated preparations of African swine fever virus in pigs_ Am j Vet Res, 1967. 28 (123): p. 475-81.] (including inactivated vaccines, naturally weakened or attenuated vaccines, etc.), new adjuvants [Blome, Sv C. Gabriel, and M. Beer, Modern adjuvants do not enhance the efficacy of an inactivated African swine fever virus vaccine preparation. Vaccine, 2014. 32 (31): p. 3879-82.] and subunit vaccines and other new vaccines [Jancovich, JK, et al., Immunization of Pigs by DNA Prime and Recombinant Vaccinia Virus Boost To Identify and Rank African Swine Fever Virus Immunogenic and Protective Proteins. J Virol, 2018. 92(8); Gomez-Puertas, P., et al., The African swine fever virus proteins p54 and p30 are involved in two distinct steps of virus attachment and both contribute to the antibody-mediated protective immune response. Virology, 1998. 243(2): p. 461-71; Neilan, JG, et al., Neutralizing antibodies to African swine fever virus proteins p30, p54, and p72 are not sufficient for antibody-mediated protection. Virology, 2004. 319 (2): p. 337-42;Ruiz-Gonzalvo/ Fv F. Rodriguez, and JM Escribano, Functional and immunological properties of the baculovirus-expressed hemagglutinin of African swine fever virus. Virology, 1996. 218(1): p. 285- 9; Lacasta, Av et al., Expression library immunization can confer protection against lethal challenge with African swine fever virus. J Virol, 2014. 88(22): p. 13322-32.] and other development strategies have been adopted, which was unsuccessful. Therefore, the disease has been plundered everywhere for many years, spreading from Africa to the world. If the culling strategy was only relied on, only a handful of countries such as Spain and Brazil have successfully controlled the African swine fever. However, because of the huge number of pigs in China, the cost is too high if the culling method is used alone. Safe and effective vaccines are urgently needed to control the African swine fever epidemic in China! Currently, attenuation through gene deletion has become an important direction of African swine fever vaccine's research.

The African swine fever is a double-stranded DNA virus with a large genome and numerous genotypes (24 genotypes) [Quembo, CJ, et al., Genetic characterization of African swine fever virus isolates from soft ticks at the wildlife/domestic interface in Mozambique and identification of a novel genotype. Transbound Emerg Dis, 2018. 65(2): p. 420-431.], which encodes 150-167 proteins. The immune escape mechanism is complex. Generally, multiple virulence genes work together, which leads to large differences in biological characteristics between different genotypes, and poor cross-immunity protection between different genotypes.

[Souto, R., et al., Vaccine Potential of Two Previously Uncharacterized African Swine Fever Virus Isolates from Southern Africa and Heterologous Cross Protection of an Avirulent European Isolate. Transbound Emerg Dis, 2016. 63 (2) : p. 224-31; King, K., et a., Protection of European domestic pigs from virulent African isolates of African swine fever virus by experimental immunisation. Vaccine, 2011. 29 (28) : p. 4593-600.]_{∘}

CN 106459931 A disclosed attenuated African swine fever virus lacking a functional form of the following genes: multigene family 360 genes 9L, 10L, 11L, 12L, 13L and 14L, and multigene family 505 genes 1R, 2R, 3R and 4R also provide attenuated African swine fever virus lacking a functional form of the DP148R gene. And the different ways of administration, such as intramuscular injection, intranasal and oral administration were also discussed.

US9474797B1 gave the full-length DNA sequence of the African swine fever virus ASF-GVAV, in which the deletion of the fragments at positions 178643 to 182578 led to the deletion of reading frames of MGF505-11L, MGF100-1L, 17L18LASFV GACD 01870, I9R, I10U11Land obtained the attenuated African swine fever virus by combining with various other mutations, US9528094B2 disclosed a mutant virus of the African swine fever virus ASF-Georgia2007, Which could produce effective protection under the dose of 10²-10⁴HAD₅₀. The mutations mainly caused partial deletion of MGF5001R and 3R genes and all deletions of MGF360 genes 12L, 13L and 14L and MGF500 2R. The WO 201/107614 A1 mainly involves the inhibition of the expression of pp220, pp62 or pB438L, and the possible deletion of the CD2 gene and the pol X gene. WO2016049101A disclosed a mutant virus ASFV-G A9GL (that is, the 172 nucleotide sequence of the 9GL gene deleted) of an African swine fever virus ASF-Georgia2007.

The preliminary study of the present invention also found that even if the same virulence gene deletion strategy was adopted, the attenuation effects of different genotypes were very different: for example, a BA71 strain (genotype I) lacking CD2V had been sufficiently attenuated, and the survival rate of pigs immunized by the BA71 strain (genotype I) lacking CD2V was 100% [Monteagudo, PL., et al., BA71DeltaCD2: a New Recombinant Live Attenuated African Swine Fever Virus with Cross-Protective Capabilities. J Virol, 2017.91 (21 ) .]_{∘} However, the survival rates of pigs immunized by the Chinese epidemic strain (genotype II) and Malawi Lil-20/1 strain (genotype II) with the same gene deletion [Borca, M_Vv et al" Deletion of a CD2-like gene, 8-DR, from African swine fever virus affects viral infection in domestic swine. J Virol, 1998. 72(4): p.2881-9]were about 50% (data not shown) and 0%. Benin 97/1 strain with DP148R deletion (genotype I) [Reis, AL, et al., Deletion of the African Swine Fever Virus Gene DP148R Does Not Reduce Virus Replication in Culture but Reduces Virus Virulence in Pigs and Induces High Levels of Protection against Challenge. J Virol, 2017. 91(24).] could also fully weaken the virus, and the survival rate of pigs immunized by the Benin 97/1 strain with DP148R deletion (genotype I) was also 100%, but the survival rate of pigs immunized by the Chinese epidemic strain laking DP148R was only 0%, and the attenuation effect is extremely limited.

The inventors isolated and obtained the Chinese epidemic strain Pig/CN/HLJ/2018. After sequencing and sequence analysis, the full-length genome sequence (GenBank: MK333180.1) and DB/LN/2018 were both 189,404 bp, while the full-length of PoL/2017 strain was 189,401 bp, and the insertion, deletion and mutation of the sequence were analyzed [Xuexia Wen et al. Genome sequences derived from pig and dried blood pig feed samples provide important insights into the transmission of African swine fever virus in China in 2018. Emerging Microbes & Infections. 2019, VOL 8]. Studies have shown that the African swine fever virus is highly virulent and infectious [Dongming Zhao et al. Replication and virulence in pigs of the first African swine fever virus isolated in China. Emerging Microbes & Infections. 2019, VOL 8]. Therefore, the development of related vaccines is urgent, we should pay particular attention to safety and effectiveness indicators.

### SUMMARY

In response to the lack of an African swine fever vaccine and the unsatisfactory effectiveness of vaccines in the prior art, especially the lack of domestic vaccines, the present invention researches and develops a method of an African swine fever gene deletion attenuated vaccine, and successfully obtains two safe and effective vaccine strains.

The present invention uses genetic engineering methods to delete virulence genes (CD2V and MGF360-505R) of the African swine fever isolated strain circulating in China, and obtains two deletion virus strains (rASF △△360-eGFP and rASFV △CD2VyS60-eGFP) -mCherry). After systematic evaluation of immunity and protection effect against poisoning, the potency and safety are excellent.

Therefore, the present invention provides a gene deletion attenuated African swine fever virus. The gene deletion attenuated African swine fever virus is obtained by deleting the MGF360-505R gene of the gene type II African swine fever virus or by jointly deleting the CD2V and MGF360-505R genes of the gene type II African swine fever virus. More preferably, the combined deletion of MGF360-505R refers to the deletion of all the two gene sequences of MGF360 and 505R, or the combined deletion of CD2V and MGF360-505R refers to the deletion of all the three gene sequences of CD2V, MGF360, and 505R.

Preferably, the genotype II African swine fever virus is a Chinese epidemic strain Pig/CN/HLJ/2018, and the full-length sequence thereof has been published in GenBank (GenBank: MK333180.1):
The preferred MGF360-505R gene deletion virus of the present invention deletes a 27942-35500th nucleotide relative to an original strain's full-length sequence. The CD2V and MGF360-505R gene combined deletion viruses delete a 27942-35500th nucleotide and a 73394-74476th nucleotide relative to the original strain's full-length sequence. A MGF360-505R gene deletion virus constructed more specifically and a gene deletion virus jointly lacking CD2V and MGF360-505R have been performed with patent procedures and deposited in the China Center for Type Culture Collection on April 11, 2019 with a conservation numbers of CCTCCNO: V201925 and CCTCCNO: V201924.

Further, the present invention also provides a vaccine comprising an attenuated African swine fever virus of the present invention, which induces a protective immune response in a subject against subsequent African swine fever virus challenge.

The vaccine can contain a plurality of attenuated African swine fever viruses of different genotypes. Such vaccines may be able to induce a cross-protective immune response against a plurality of ASF virus genotypes.

The present invention also provides a pharmaceutical composition containing one or more of attenuated African swine fever viruses of the present invention. The pharmaceutical composition can be used to prevent or treat African swine fever. The vaccine or the pharmaceutical composition may comprise one or more of the attenuated African swine fever virus of the present invention, and further optionally comprise one or more adjuvants, excipients, carriers and diluents. The adjuvant can be any suitable adjuvant, chemical immune adjuvants such as aluminum hydroxide, Freund's adjuvant, mineral oil, Span, etc.; microbial immune adjuvants such as mycobacteria, BCC, lipopolysaccharide,muramyl dipeptide, cytosines, fat-soluble waxiness D, Corynebacterium parvum; plant immune adjuvants such as polysaccharides mostly extracted from plants or large fungi, such as tuckahoe polysaccharides, safflower polysaccharides, and Chinese herbal medicines. And biochemical immune adjuvants include thymosin, transfer factor, interleukin and so on. Preferred adjuvants can be nano adjuvant biological adjuvants, interleukins, interferons and the like.

The vaccine of the present invention can also be used in combination vaccines, such as in combination with other vaccines for pigs, but the focus is on live attenuated vaccines, especially the integration of viral genes, such as bivalent vaccines, trivalent vaccines, and the like. The combination vaccine can contain a plurality of attenuated swine fever viruses of different genotypes so that a cross-protective immune response against a plurality of African swine fever virus genotypes can be induced.

The vaccine of the present invention can be administered in convenient ways, such as intramuscular injection, intranasal administration, oral administration, subcutaneous administration, transdermal administration and vaginal administration. The attenuated vaccine of the present invention is preferably injected intramuscularly with the appropriate dose of 10^{2.5}-10^{2.5} TCID₅₀, and a preferred dose of 10³-10³ TCID₅₀. The vaccine can be administered after a prime-boost solution.

For example, after the first vaccination, a subject may receive a second boost administration after a period of time (eg, about 7, 14, 21, or 28 days). Generally, the dose for boost administration is the same or lower than the dose for priming administration. In addition, a third boost immunization can be performed, for example, 2-3 months, 6 months, or one year after immunization.

The gene delection attenuated African swine fever virus obtained in the present invention has the following advantages. After pigs are immunized by two strains of gene delection African swine fever virus (rASFV △ 360-eGFP and rASFV △CD2Vy360-eGFP-mCherry) constructed by the present invention, even if the animals in the immunized group at a dose of 10⁵ TCID⁵⁰ has no clinical symptoms such as elevated body temperature, blood and organ virus are isolated to be negative, and the survival rate is 100%, indicating that the gene deletion African swine fever virus is sufficiently weakened, is very safe as a vaccine, and is superior to similar studies of foreign genotype II ASFV [Borca, MV, et al., Deletion of a CD2-like gene, 8-DR, from African swine fever virus affects viral infection in domestic swine. J Virol, 1998. 72(4): p. 2881-9; O'Donnell, Vv et al., African Swine Fever Virus Georgia Isolate Harboring Deletions of MGF360 and MGF505 Genes Is Attenuated in Swine and Confers Protection against Challenge with Virulent Parental Virus. J Virol, 2015. 89 (11): p. 6048-56.] (Table 1): a Georgia strain with high homology of China is used to construct MGF360-505R gene deletion virus abroad, and the positive rate of blood virus isolation of the immunized pigs is over 60%. The positive rate of blood virus isolation of pigs immunized by the CD2V single gene deletion virus constructed by a gene type II Malawi Lil-20/1 strain is 100%, and the survival rate is only 0%. Secondly, in terms of effectiveness, after live challenge, rASFV △360-eGFP and rASFV △CD2V/360-eGFP-mCherry constructed in the present invention have an immunological protection rate of 100%, and the positive rate of blood virus isolation is less than 35% and 100%, respectively, but on 21th-22th days, both turn negative, indicating that the two strains have excellent immune protection efficacy, and are also significantly better than similar foreign studies using genotype II ASFV [Borca, MV, et al_, Deletion of a CD2-like gene, 8-DR, from African swine fever virus affects viral infection in domestic swine. J Virol, 1998. 72(4): p. 2881-9; O'Donnell, Vv et alv African Swine Fever Virus Georgia Isolate Harboring Deletions of MGF360 and MGF505 Genes Is Attenuated in Swine and Confers Protection against Challenge with Virulent Parental Virus. J Virol, 2015. 89 (11): p. 6048-56.] (Table 1): The positive rate of blood virus isolation of pigs immunized by Foreign MGF360-505R lacking Georgia strain after challenge is 70% [O'DonneN/ V., et al., African Swine Fever Virus Georgia Isolate Harboring Deletions of MGF360 and MGF505 Genes Is Attenuated in Swine and Confers Protection against Challenge with Virulent Parental Virus. J Virol, 2015. 89 (11): p. 6048-56] (the 10³ TCID₅₀ immunization dose used in the present invention is less than the 10⁴ HAD₅₀ abroad, but the positive rate of blood virus isolation after challenge is only 25%). Although the positive rate of blood virus isolation of pigs immunized by the MGF360-505R and CD2V double gene deletion virus constructed by the present invention after the challenge is 100%, which is slightly worse than the MGF360-505R gene deletion virus, but significantly better than the CD2V single deletion genotype II Ma lawi Lil-20/1 strain (100% mortality after immunization) [Borca, MV, et al., Deletion of a CD2-like gene, 8-DR, from African swine fever virus affects viral infection in domestic swine, J Virol, 1998. 72(4): p. 2881-9], and the blood virus isolation turns negative 21 days after the challenge. Finally, the two strains of gene deletion viruses constructed by the present invention can provide sufficient protection against live challenge 21 days after being immunized, which is better than 28 days abroad [O'Donnell, V., et al., African Swine Fever Virus Georgia Isolate Harboring] [O'Donnell, V., et al., African Swine Fever Virus Georgia Isolate Harboring] Deletions of MGF360 and MGF505 Genes Is Attenuated in Swine and Confers Protection against Challenge with Virulent Parental Virus. J Virol, 2015. 89(11): p.6048-56]. Therefore, it is the best strategy to use isolated strains from endemic areas to construct an attenuated African swine fever gene deletion vaccine, and it is also the key to China's development of an African swine fever vaccine. Therefore, the gene-deficient African swine fever virus (rASFV △360-eGFP and rASFV △CD2V/360-eGFP-mCherry) constructed by the present invention has great application value.

**Table 1 Comparison of the safety of similar gene deletion attenuated African swine fever virus (genotype II) at home and abroad**

| | Foreign reports | | The present invention | |
|---|---|---|---|---|
| Deleted virulence gene | MGF360-505R[20] | CD2V[19] | MGF360-505R | CD2V and MGF360-505R |
| Strains and genotypes | Georgia strain | Malawi Lil-20/1 Strains | CN / HLJ / 18 strain | CN / HLJ / 18 strain |
| Genotype | Genotype II | Genotype II | Genotype II | Genotype II |
| Positive rate of virus isolation after immunization | ≥60% | 100% | 0% | 0% |
| Survival rate after immunization | 100% | 0% | 100% | 100% |
| Positive rate of blood virus isolation after challenge | 70% (10⁴ HAD₅₀ dose immunity), | - | 25% (10³ TCID₅₀ dose immunity) | 100% (10³ TCID₅₀ dose immunity) |
| Survival rate after challenge | 100% | - | 100% | 100% |

| | | | | |
|---|---|---|---|---|
| Note: "--" means no challenge (the immunization of this strain causes all pigs to die). | | | | |

Conservation information: The specific virus based on the CN/HU/18 strain of the MGF360-505R gene deletion and the specific virus with the combined deletion of CD2V and MGF360-505R of the present invention have been performed with patent procedures and deposited on April 11, 2019 in the China Center for Type Culture Collection with a conservation number of CCTCC NO:V201925 and CCTCCNO:V201924.

### BRIEF DESCRIPTION OF THE DIAGRAMS

FIG. 1 shows a diagram of construction of a carrier for CD2V gene deletion.
FIG. 2 shows a diagram of construction of a carrier (expressing green fluorescence) for MGF360-505R gene deletion.
FIG. 3 shows a diagram of construction of a carrier (expressing red fluorescence) for MGF360-505R gene deletion.
FIG. 4 shows a diagram that African swine fever gene deletion virus expresses green or red and green fluorescent protein.
FIG. 5 shows purification and identification of African swine fever gene deletion virus.

Where, △CD2V is rASFV △CD2V-eGFP, △360 is rASFV△360-eGFP, and CD2V/360 is rASFV △ CD2V/360-eGFP-mCherry.
FIG. 6 shows changes in body temperature of rASFV△360eGFP and rASFV△CD2V/360-eGFP-mCherry immunized pigs and cohabiting pigs.
FIG. 7 shows changes in body temperature of rASFV△360-eGFP and rASFV△CD2V/360-eGFP-mCherry immunized pigs and cohabiting pigs after challenge.
FIG. 8 shows a survival rate of test pigs after immunization or challenge.

Where, △CD2V is rASFV△CD2V-eGFP, △360 is rASFV△360-eGFP, and CD2V/360 is rASFV△ CD2V/360-eGFP-mCherry.

### DETAILED DESCRIPTION

The present invention will be further described below through specific implementations for a better understanding of the present invention, but it does not constitute a limitation to the present invention.

### Materials and methods used in embodiments

Chinese epidemic strain of African swine fever virus (ASFV/CN/HU/18, also known as the Chinese epidemic strain of African swine fever virus Pig/CN/HLJ/2018) was isolated and saved by Harbin Veterinary Research Institute, (the full-length sequence is in GenBank: MK333180.1): Primary porcine alveolar macrophages (Primary porcine alveolar macrophages, PAM) are derived from SPF pigs aged 30-50 days in 1640 medium containing 10% FBS (purchased from Thermo Scientific, USA). Peripheral Blood Mononuclear Cell (PBMC) is isolated from SPF pig EDTA anticoagulant blood using a PBMC isolation kit (purchased from China TBD company).

The operations in the experiment are all known in the art unless otherwise specified.

### Embodiment 1. Homologous recombination carrier construction

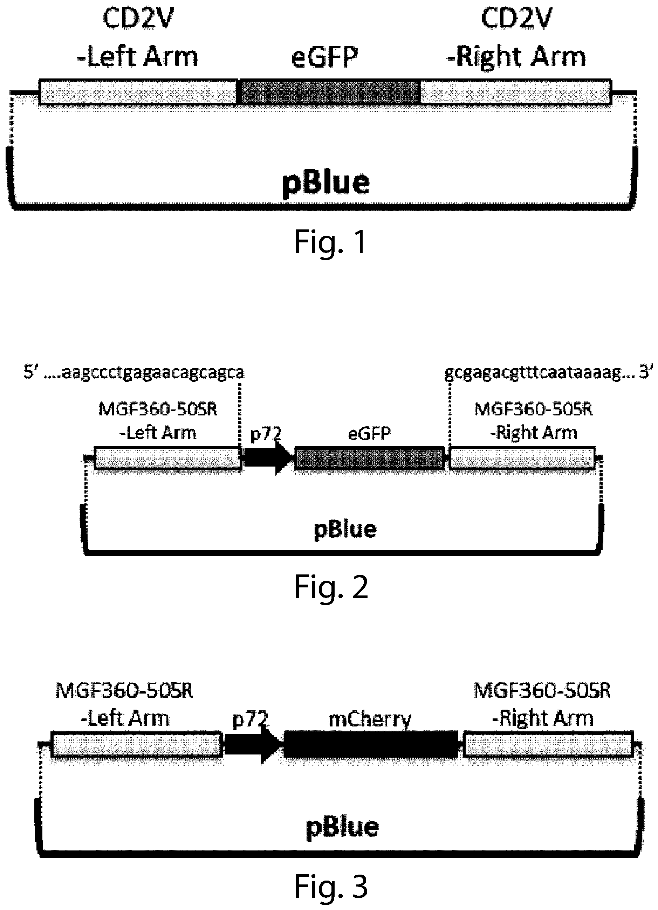

About 1000 bp of the genome on the left and right sides of CD2V gene ORF (as left and right homology arms, the sequence position is the 73394-74476th relative to a full-length sequence) and a PCR fragment of eGFP with a one-step cloning kit (purchased from China Vazyme Biotech Co., Ltd.) was cloned into a pBluescripdlKS(+) carrier, in which the eGFP gene was expressed using a promoter of a CD2V gene itself, and a plasmid pB-LR△CD2V-eGFP was obtained (FIG. 1).

PCR fragments of four genes including the left and right homology arms of MGF360-505R (about 1000 bp to a left side of the 5'-aagccctgagaacagcagca-3' sequence of an ASF genome and about 1000 bp to a right side of the 5'-gcgagacgtttcaataaaag-3' sequence of the ASF genome) (in a 27942-35500th position relative to the full-length sequence), p72 promoter [Reis, AL, et al., Deletion of the African Swine Fever Virus Gene DP148R Does Not Reduce Virus Replication in Culture but Reduces Virus Virulence in Pigs and Induces High Levels of Protection against Challenge. J Virol, 2017. 91(24)] and eGFP were cloned into a pBluescriptllKS(+) carrier with a one-step cloning kit, wherein the eGFP gene was expressed under the control of the p72 promoter to obtain a plasmid pB-LR △360-eGFP (FIG. 2). The eGFP gene in pB-LR △360-eGFP is replaced into a mCherry gene to obtain the plasmid pB-LR △360-mCherry (FIG. 3).

### Embodiment 2: Construction, purification and identification of gene deletion African swine fever virus

The plasmids pB-LR △360-eGFP and pB-LR △360-mCherry were transfected into PAM cells infected with ASFV with TransIT-LTl transfection reagent (purchased from Mirus Bio, USA). CD2V was purified with a scab cloning method. MGF360-505R genes were deleted respectively. African swine oncogene deletion virus that expressed green fluorescent protein at the same time [Reis, AL, et al., Deletion of the African Swine Fever Virus Gene DP148R Does Not Reduce Virus Replication in Culture but Reduces Virus Virulence in Pigs and Induces High Levels of Protection against Challenge. J Virol, 2017. 91(24).; Chen, Wv et a\.f A goat poxvirus-vectored peste-des-petits-ruminants vaccine induces long-lasting neutralization antibody to high levels in goats and sheep. Vaccine, 2010. 28(30): p. 4742-50.; Chen Weiye, et al., Research on recombinant goat pox virus vaccine expressing the H protein of Peste des petits ruminants. Chinese Journal of Biological Engineering, 2009. 25 ( 4): p. 496-502.] was named as rASFV △CD2V-eGFP and rASFV △360-eGFP (a conservation number is CCTCCNO:V201925), respectively. Relative to the full-length sequence of the Chinese epidemic strain of African swine fever Pig/CN/HLJ/2018, the former deleted a 73394-74476th nucleotide, and the latter deleted a 27942-35500th nucleotide.

Similarly, the homologous recombination plasmid pB-LR △360-mCherry was transfected into rASFV△ CD2V-eGFP-infected PAM cells according to the above method. the virus with double deletion of CD2V and MGF360-505R genes obtained through multiple rounds of plaque purification was named as rASFV △ CD2V/360-eGFP-mCherry (a conservation number is CCTCCNO:V201924). Relative to the full-length sequence of the Chinese epidemic strain of African swine fever Pig/CN/HLJ/2018, the 73394-74476th nucleotide and the 27942-35500th nucleotide were deleted at the same time.

Genomes of ASFV and three gene-deleted ASFV were extracted with a viral genome extraction kit (purchased from Tiangen Biotech (Beijing) Co., Ltd.). CD2V (5'-CACCACCTGAATCTAATGAAG-3' and 5,-GCGGGATATTGGGTAGTAG-3,) and MGF360 -505R (5,-CGTCTATTTGGATGTTTT-3, and 5'-CGGCAGTATTATTTTGTG-3') primers were performed with PCR identification respectively to confirm whether the deletion is successful.

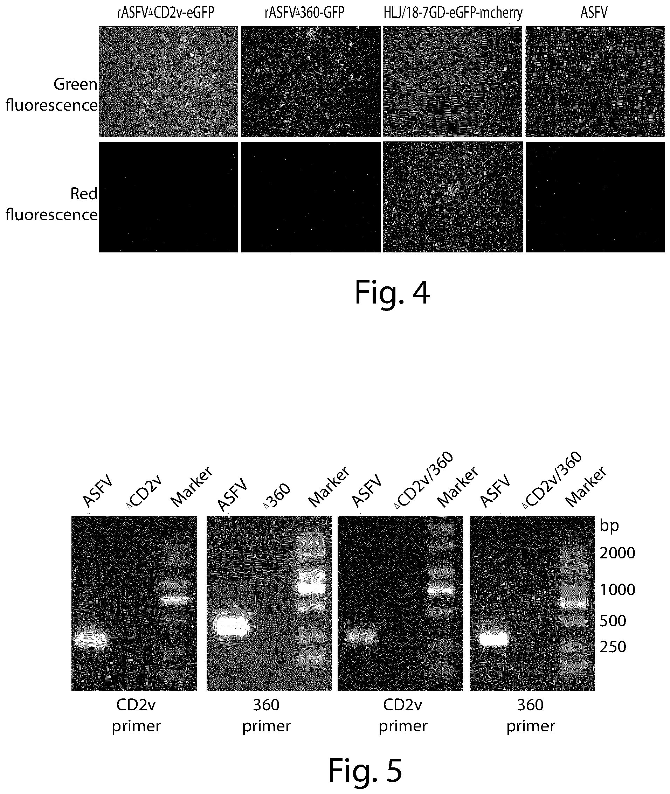

The transfected and purified gene deletion viruses rASFV △CD2V-eGFP, rASFV △360-eGFP, and rASFV △CD2V/360-eGFP-mCherry expressed green fluorescence, green fluorescence, red and green fluorescences in PAM cells respectively (FIG. 4). The deletion genes of three viruses were identified with PCR, and the results (FIG. 5) showed that the ASFV control all amplified CD2V and MGF360 gene fragments, while the gene deletion viruses did not amplify the corresponding deleted genes.

### Embodiment 3: Titration of virus titer

The titration of African swine fever virus was performed using two methods: half of the cell infection dose (50% Tissue Culture Infectious Dose, TGD50) and half of blood cell adsorption (50% haemadsorption, HAD₅₀).

The TCID₅₀ titration was carried out according to the following steps: ASFV is then diluted continuously by 10 times in serum-free 1640 medium. PAM cells cultured in a 96-well culture plate with a density of about 90-100%- were inoculated. 8 wells of 0.02mL were inoculated for each dilution. After 1 hour incubation at 37°C and 5% concentration CO₂, 0.1mL 1640 complete culture medium containing 10% fetal bovine serum was added to each well, cultured at 37°C, 5% concentration CO₂, and observe for 3-7 days. A half-cell infection amount (TCID₅₀) was calculated according to cytopathic or green fluorescence and Reed and Muench methods [4].

The HAD₅₀ test was operated according to the literature [5] and adjusted appropriately: the primary PBMC was inoculated in a 96-well cell culture plate. a 10-fold dilution of a sample to be tested was performed. 0.02ml was inoculated per well. Viral infection could be determined according to rosettes formed by red blood cells around the infected cells and observed for 7 days. Half of blood cells adsorbed dose (HAD₅₀) were calculated according to the Reed and Muench method [Reed, L. and H. Muench, A simple method of estimaing fifty percent endpoints. American Journal of Epidemiology 1938. 27: p. 493-497].

### Embodiment 4: Animal Immunization Experiment

7-week-old Dabai and Changbai inbred line SPF pigs (purchased from the Animal Center of Harbin Veterinary Research Institute, CAAS) were introduced into the Biosafety Level 4 Laboratory Animal Room of Harbin Veterinary Research Institute, CAAS. After the pigs were adapted for 2-3 days, the pigs were grouped and immunized according to Table 2-1. In addition, a test for organ virus distribution was designed (Table 2-2). After immunization, the animal's mental status and feeding status were continue to observe, the animal's body temperature was monitored, EDTA anticoagulant blood was collected and serum was separated. 11

**Table 2-1 Test grouping**

| Virus | Dose | Immune pathway | Quantity and number |
|---|---|---|---|
| rASFV △360-eGFP immune group | 10³ and 10⁵ TCID₅₀ | Intramuscular injection (IM) | 4 per dose, No. 90-93 and No. 95-98 |
| rASFV △ CD2V/360-eGFP-mCherry immune group | 10³ and 10⁵ TCID₅₀ | Intramuscular injection (IM) | 4 per dose, No. 80-83 and No. 85-88 |
| Cohabitation infection group | - | - | 2 pigs , No. 101 and No. 102 |

**Table 2-2 Organ toxicity test after immunization**

| Virus | Dose | Immune pathway | Quantity and number |
|---|---|---|---|
| rASFV △360-eGFP immune group | 10⁵ | Intramuscular injection (IM) | Dissection was performed at 5, 10, 15, and 21 days, organs were taken to isolate the virus, 4 pigs at each time point, No. 121-136 |
| rASFV CD2V/360-eGFP-mCherry immune group | 10⁵ | Intramuscular injection (IM) | Dissection was performed at the 5th, 10th, 15th, and 21th days, organs were taken to isolate the virus, 4 pigs at each time point, No. 137-152 |

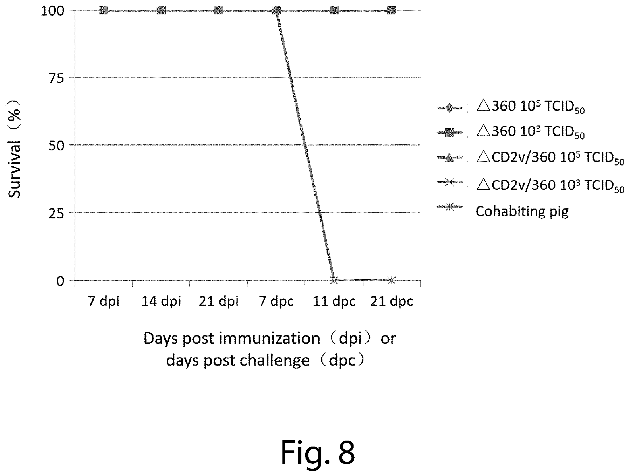

The body temperature test after immunization showed that after the pigs were immunized with rASFV △ 360-eGFP, either a high-dose (105 TCID50, No. 90-93) immunization group or a low-dose immunization group (10³ TCID₅₀, No. 95-98) had no significant increase in body temperature, and the survival rate was 100% (FIG. 8). Pig No. 90 died accidentally on the 16th day. There was no obvious pathological change in the organs after autopsy, and blood poisoning results were negative. After pigs were immunized with rASFV △ CD2V/360-eGFP-mCherry , there was no increase in body temperature in either the high-dose (10⁵ TCID₅₀, No. 80-83) immunization group or the low-dose immunization group (10³ TCID₅₀, No. 85-88). In addition, cohabiting pigs (No. 101 and No. 102) also showed no increase in body temperature. The immunized pigs and cohabiting pigs have no abnormal mental status and feeding status after immunization, and no obvious clinical symptoms

The results of virus isolation in blood samples after immunization (Table 3-1) showed that virus was not isolated for rASFV △360-eGFP, rASFV△CD2V/360-eGFP-mCherry immunized pigs and cohabiting pigs at the 5th, 9th, 14th, 19th days after immunization.

In addition, all pigs immunized with rASFV △360-eGFP, rASFV△CD2V/3360-eGFP-mCherry had negative virus isolation in each organ within 21 days after immunization. At the same time, there were no abnormal phenomena in mental status, feeding status and clinical symptoms (Table 3-2).

In summary, the two gene deletion strains of rASFV △360-eGFP and rASFV△CD2V/360-eGFP-mCherry were sufficiently attenuated in pigs.

**Table 3-1 blood virus isolation and detection of pigs immunized with rASFV △360-eGFP and rASFV△ CD2V/360-eGFP-mCherry**

| Strain and immune dose | Num ber. | Blood poisoning | | | |
|---|---|---|---|---|---|
| | | 5 days | 9 days | 14 days | 19 days |
| rASFV △360-eGFP, 10⁵ TCID₅₀ | 90 | N | N | N | / |
| | 91 | N | N | N | N |
| | 92 | N | N | N | N |
| | 93 | N | N | N | N |
| rASFV △360-eGFP, 10³ TCID₅₀ | 95 | N | N | N | N |
| | 96 | N | N | N | N |
| | 97 | N | N | N | N |
| | 98 | N | N | N | N |
| rASFV △ CD2V/360-eGFP-mCherry, 105 TCID50 | 80 | N | N | N | N |
| | 81 | N | N | N | N |
| | 82 | N | N | N | N |
| | 83 | N | N | N | N |
| rASFV △ CD2V/360-eGFP-mCherry, 103 TCID50 | 85 | N | N | N | N |
| | 86 | N | N | N | N |
| | 87 | N | N | N | N |
| | 88 | N | N | N | N |
| Cohabitation | 101 | N | N | N | N |
| | 102 | N | N | N | N |

| | | | | | |
|---|---|---|---|---|---|
| Note: "N" means the test result was negative; and "/" means no sample. | | | | | |

**Table 3-2 Organ virus distribution of pigs immunized with rASFV △360-eGFP and rASFV△CD2V/360-eGFP-mCherrv**

| Virus strain | time (day) | Numb er | Thym us | gland ulae bronc hiales | Colon | Liver | Splee n | Duod enum | Lung | Fundu s of stoma ch | Jejun um | Kidne y | Tonsil | Intesti nal lymph node | Abdo minal cavity lymph | heart | Subm andib ular lymph atic nodes | Ileum | Gastr ohepa tic lymph node |
|---|---|---|---|---|---|---|---|---|---|---|---|---|---|---|---|---|---|---|---|
| rASFV △ 360-eGFP | 5 | 121 | N | N | N | N | N | N | N | N | N | N | N | N | N | N | N | N | N |
| | | 122 | N | N | N | N | N | N | N | N | N | N | N | N | N | N | N | N | N |
| | | 123 | N | N | N | N | N | N | N | N | N | N | N | N | N | N | N | N | N |
| | | 124 | N | N | N | N | N | N | N | N | N | N | N | N | N | N | N | N | N |
| | 10 | 125 | N | N | N | N | N | N | N | N | N | N | N | N | N | N | N | N | N |
| | | 126 | N | N | N | N | N | N | N | N | N | N | N | N | N | N | N | N | N |
| | | 127 | N | N | N | N | N | N | N | N | N | N | N | N | N | N | N | N | N |
| | | 128 | N | N | N | N | N | N | N | N | N | N | N | N | N | N | N | N | N |
| | 15 | 129 | N | N | N | N | N | N | N | N | N | N | N | N | N | N | N | N | N |
| | | 130 | N | N | N | N | N | N | N | N | N | N | N | N | N | N | N | N | N |
| | | 131 | N | N | N | N | N | N | N | N | N | N | N | N | N | N | N | N | N |
| | | 132 | N | N | N | N | N | N | N | N | N | N | N | N | N | N | N | N | N |
| | 21 | 133 | N | N | N | N | N | N | N | N | N | N | N | N | N | N | N | N | N |
| | | 134 | N | N | N | N | N | N | N | N | N | N | N | N | N | N | N | N | N |
| | | 135 | N | N | N | N | N | N | N | N | N | N | N | N | N | N | N | N | N |
| | | 136 | N | N | N | N | N | N | N | N | N | N | N | N | N | N | N | N | N |
| rASFV △ CD2V/360-eGFP-mCh | 5 | 137 | N | N | N | N | N | N | N | N | N | N | N | N | N | N | N | N | N |
| | | 138 | N | N | N | N | N | N | N | N | N | N | N | N | N | N | N | N | N |
| | | 139 | N | N | N | N | N | N | N | N | N | N | N | N | N | N | N | N | N |
| | | 140 | N | N | N | N | N | N | N | N | N | N | N | N | N | N | N | N | N |
| erry | 10 | 141 | N | N | N | N | N | N | N | N | N | N | N | N | N | N | N | N | N |
| | | 142 | N | N | N | N | N | N | N | N | N | N | N | N | N | N | N | N | N |
| | | 143 | N | N | N | N | N | N | N | N | N | N | N | N | N | N | N | N | N |
| | | 144 | N | N | N | N | N | N | N | N | N | N | N | N | N | N | N | N | N |
| | 15 | 145 | N | N | N | N | N | N | N | N | N | N | N | N | N | N | N | N | N |
| | | 146 | N | N | N | N | N | N | N | N | N | N | N | N | N | N | N | N | N |
| | | 147 | N | N | N | N | N | N | N | N | N | N | N | N | N | N | N | N | N |
| | | 148 | N | N | N | N | N | N | N | N | N | N | N | N | N | N | N | N | N |
| | 21 | 149 | N | N | N | N | N | N | N | N | N | N | N | N | N | N | N | N | N |
| | | 150 | N | N | N | N | N | N | N | N | N | N | N | N | N | N | N | N | N |
| | | 151 | N | N | N | N | N | N | N | N | N | N | N | N | N | N | N | N | N |
| | | 152 | N | N | N | N | N | N | N | N | N | N | N | N | N | N | N | N | N |

| | | | | | | | | | | | | | | | | | | | |
|---|---|---|---|---|---|---|---|---|---|---|---|---|---|---|---|---|---|---|---|
| Note: "N" means the test result is negative. | | | | | | | | | | | | | | | | | | | |

### Embodiment 5: Animal challenge test

After 21 days of immunization, all pigs in the rASFV △360-eGFP and rASFV△CD2V/360-eGFP-mCherry immunized group were challenged with ASFV virulent virus with a challenge dose of 103.5 HAD50 and a route of challenge of intramuscular injection, and two 8-weeks-old SPF pigs were introduced as challenge control at the same time. After the challenge, the animal's mental status and feeding status were continue to observe, the animal's body temperature was monitored, EDTA anticoagulant blood was collected and serum was separated.

Cohabiting pigs (No. 101 and No. 102) immunized with the rASFV △360-eGFP and rASFV △ CD2V^60-eGFP-mCherry died on the 11th day after the challenge, while the immunized group had a survival rate of 100% after the challenge (FIG. 8) until slaughter and necropsy were performed at the 21th-22th days The specific body temperature and blood toxicity were as follows.

### 1. Body temperature changes and clinical symptoms after challenge

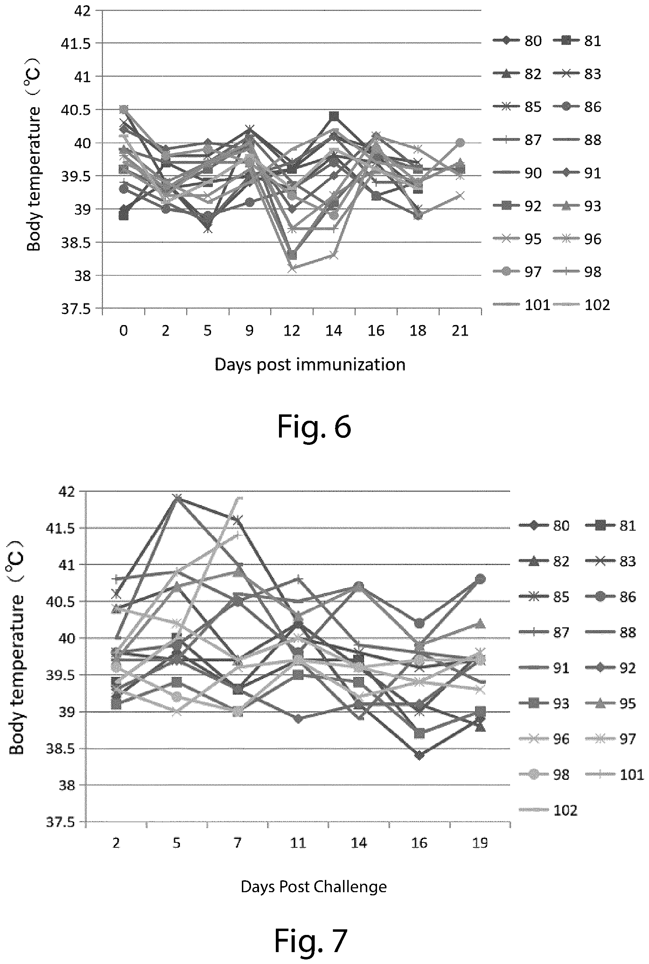

Cohabiting pigs (No. 101 and No. 102) showed clinical symptoms such as increased body temperature (40.5-42°C) (FIG. 7), depression, and decreased food intake on the 5th to 7th days after challenge.

After the pigs immunized with rASFV △360-eGFP were challenged, only pig No. 95 had a significant increase in body temperature on the 7th day.

After returning to the normal, mental status and feeding status also returned to the normal. other pigs had no abnormal body temperature, mental status and feeding status, and no clinical symptoms.

After the pigs immunized with ASFV△CD2V/360-eGFP-mCherry were challenged, only the 82th pig in a high-dose group (105 TCID50) had a transient increase in body temperature of 40.7°C on the 5th day, and then returned to the normal. The 85th, 87th, and 88th pigs in a low-dose group ( 103 TCID50) all had a transient increase in body temperature of above 41°C on the 5th-11th days, and then returned to the normal. The 86th pig had slightly higher temperature at outer body which was lower than 41°C. Pigs with elevated body temperature had a slight depression and a slight decrease in food intake, but as the body temperature of the pigs returned to the normal, the spirit and food intake returned to the normal, and there were no other obvious clinical symptoms.

### 2. Blood virus separation and test after challenge

The results of blood virus isolation (Table 4) showed that after the challenge, cohabiting pigs (No. 101 and No. 102) turned positive on the 5th day after the challenge, and died on the 7th day; however, after the pigs immunizewd with rASFV △360-eGFP were challenged, virus isolation of only 2/7 pigs (No. 91 and No. 95) were positive after the challenge. On the 5th day after the pigs immunized with rASFV △ CD2V/360-eGFP-mCherry were challenged, all the pig virus isolations turned positive, but all turned negative on the 22nd day. These results indicated that the pigs immunized with rASFV △360-eGFP and rASFV △ CD2V/360-eGFP-mCherry could provide adequate immune protection against live challenge.

**Table 4: Blood virus isolation and detection of pigs immunized wiht rASFV △360-eGFP and rASFV△ CD2V/360-eGFP-mCherry after challenge.**

| Strain and immune dose | Num ber. | Blood poisoning | | | |
|---|---|---|---|---|---|
| | | 5 days | 11 days | 16 days | 21 days |
| | | | | | |
| rASFV △360-eGFP, 105 TCID50 | 91 | + | + | + | N |
| | 92 | N | N | N | N |
| | 93 | N | N | N | N |
| rASFV △360-eGFP, 103 TCID50 | 95 | + | + | + | N |
| | 96 | N | N | N | N |
| | 97 | N | N | N | N |
| | 98 | N | N | N | N |
| rASFV△CD2V^60-eGFP-mCherry, 105 TCID50 | 80 | + | N | N | N |
| | 81 | + | N | N | N |
| | 82 | + | + | + | N |
| | 83 | + | N | N | N |
| rASFV△CD2V^60-eGFP-mCherry, 103 TCID50 | 85 | + | + | + | N |
| | 86 | + | + | + | N |
| | 87 | + | + | + | N |
| | 88 | + | + | + | N |
| Cohabitation | 101 | + | / | / | / |
| | 102 | + | / | / | / |

| | | | | | |
|---|---|---|---|---|---|
| Note: "+" means the test result is positive; "N" means the test result is negative. | | | | | |

## Claims

1. A gene deletion attenuated African swine fever virus, **characterized in that** a MGF360-505R gene of a genotype II African swine fever virus is deleted, or CD2V and MGF360-505R genes jointly lack the virus, the genotype II African swine fever virus is a full-length genome sequence as described Chinese epidemic strain Pig/CN/HLJ/2018 in GenBank: MK333180.1

2. The gene deletion attenuated African swine fever virus according to claim 1, **characterized in that** the MGF360-505R gene deletion virus deletes a 27942-35500th nucleotide relative to a full-length sequence of the original strain.

3. The gene deletion attenuated African swine fever virus according to claim 2, **characterized in that** the MGF360-505R gene deletion virus has a conservation number of CCTCC NO: V201925.

4. The gene deletion attenuated African swine fever virus according to claim 1, **characterized in that** the CD2V and MGF360-505R gene combined deletion virus delete the 27942-35500th nucleotide and a 73394-74476th nucleotide.

5. The gene deletion attenuated African swine fever virus according to claim 4, **characterized in that** the CD2V and MGF360-505R gene combined deletion virus has a conservation number of CCTCC NO: V201924.

6. An African swine fever vaccine, **characterized by** containing a gene deletion attenuated African swine fever virus according to any one of claims 1 to 5.

7. The African swine fever vaccine according to claim 6, **characterized in that** nano adjuvant biological adjuvant, interleukin, or interferon is first used as an adjuvant.

8. The African swine fever vaccine of claim 6, **characterized in that** a combination vaccine is prepared with other vaccines.

9. A method for preparing a gene deletion attenuated African swine fever virus according to any one of claims 1 to 5, **characterized in that** a MGF360-505R gene sequence of an original strain, or CD2V and MGF360-505R gene sequence are jointly deleted by genetic engineering means to prepare the gene deletion attenuated African swine fever virus.


Sequence

<110>HARBIN VETERINARY RESEARCH INSTITUTE,CHIINESE ACADEMY OF AGRICULTRUAL
SCIENCES
<120>ATTENUATED AFRICAN AWINE FEVER VIRUS WITH DELETED GENE AND USE OF SAME
AS VACCINE
<160>6

<210>1
<211>20
<212>DNA
<213>PRT

<400> 1
aagcc ctgag aacag cagca     20

<210>2
<211>20
<212>DNA
<213>Artificaial sequence

<400>2
gcgag acgtt tcaat aaaag    20

<210>3
<211>21
<212>DNA
<213>Artificaial sequence

<400>3
CACCA CCTGA ATCTA ATGAA G     21

<210>4
<211>19
<212>DNA
<213>Artificaial sequence

<400>4
GCGGG ATATT GGGTA GTAG    19

<210>5
<211>18
<212>DNA
<213>Artificaial sequence

<400>5
CGTCTATTTGGATGTTTT    18

<210>6
<211>18
<212>DNA
<213>Artificaial sequence

<400>6
CGGCA GTATT ATTTT GTG    18